(19) Europäisches Patentamt
European Patent Office
Office européen des brevets

(11) Publication number: **0 079 675**
**A1**

(12) # EUROPEAN PATENT APPLICATION

(21) Application number: 82305385.5

(22) Date of filing: 08.10.82

(51) Int. Cl.³: C 07 D 498/10
A 61 K 31/42
//(C07D498/10, 263/00, 209/00)

(30) Priority: 13.10.81 US 310682

(43) Date of publication of application:
25.05.83 Bulletin 83/21

(84) Designated Contracting States:
AT BE CH DE FR GB IT LI LU NL SE

(71) Applicant: PFIZER INC.
235 East 42nd Street
New York, N.Y. 10017(US)

(72) Inventor: Hutchison, Alan Jeffrey
33 Wilson Avenue
Kearny New Jersey(US)

(74) Representative: Moore, James William
Pfizer Limited Ramsgate Road
Sandwich Kent CT13 9NJ(GB)

(54) **Oxazolidinedione derivatives.**

(57) A series of novel *spiro*-oxindole oxazolidinedione derivatives have been prepared, including their base salts with pharmacologically acceptable cations. These compounds are useful in therapy as aldose reductase inhibitors for the control of certain chronic diabetic complications. Preferred compounds include 1-(*p*-fluorophenyl)-5-fluoro-*spiro*-[indoline-3,5'-oxazolidine]-2,2',4'-trione, 1-methyl-5-chloro-*spiro*-[indoline-3,5'-oxazolidine]-2,2',4'-trione, 1-isopropyl-5-chloro-*spiro*-[indoline-3,5'-oxazolidine]-2,2',4'-trione and 1-(*p*-chlorobenzyl)-5-chloro-*spiro*-[indoline-3,5'-oxazolidine]-2,2'-4'-trione. Methods for preparing these compounds from known starting materials are provided.

EP 0 079 675 A1

P. C. 6447

OXAZOLIDINEDIONE DERIVATIVES

This invention relates to new oxazolidinedione derivatives of interest to those in the field of medicinal chemistry and chemotherapy. More particularly, it is concerned with a novel series of spiro-oxindole oxazolidinedione compounds for the control of certain chronic complications arising from diabetes mellitus (e.g., diabetic cataracts, retinopathy and neuropathy).

Past attempts to obtain new and better oral antidiabetic agents have, for the most part, involved an endeavor to lower blood sugar levels. However, little is known about the effect of organic compounds in preventing or arresting certain chronic complications of diabetes, such as diabetic cataracts, neuropathy and retinopathy, etc. Nevertheless, K. Sestanj et al. in U.S. Patent No. 3,821,383 do disclose that certain aldose reductase inhibitors like 1,3-dioxo-1H-benz[d,e]isoquinoline-2(3H)-acetic acid and some closely-related derivatives thereof are useful for these purposes even though they are not known to be hypoglycemic. These compounds function by inhibiting the activity of the enzyme aldose reductase, which is primarily responsible for catalyzing the reduction of aldoses (like glucose

and galactose) to the corresponding polyols (such as sorbitol and galactitol) in the human body. In this way, unwanted accumulations of galactitol in the lens of galactosemic subjects and of sorbitol in the lens, retina, peripheral nervous system and kidney of diabetic subjects are thereby prevented or reduced. As a result, these compounds control certain chronic diabetic complications, including those of an ocular nature, since it is already known in the art that the presence of polyols in the lens of the eye leads to cataract formation and concomitant loss of lens clarity.

The present invention relates to novel spiro-oxindole oxazolidinedione compounds useful in therapy as aldose reductase inhibitors for the control of certain chronic complications arising in a diabetic subject. More specifically, the novel compounds of this invention are selected from the group consisting of spiro-oxindole oxazolidinedione derivatives of the formula:

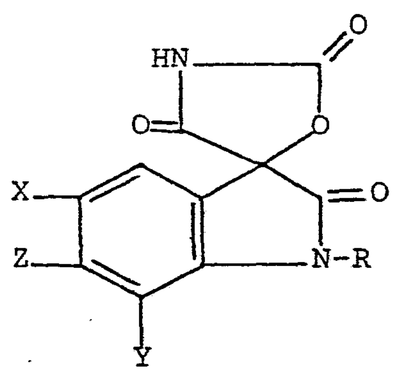

and the base salts thereof with pharmacologically acceptable cations, wherein X and Y are each hydrogen, fluorine, chlorine, bromine, nitro or amino; Z is hydrogen or amino, with the proviso that Z is hydrogen when at least one of X and Y is other than hydrogen; and R is hydrogen, alkyl having from one to four carbon atoms, aryl or aralkyl having up to three carbon atoms

in the alkyl moiety wherein each of said aryl moieties is naphthyl, pyridyl, furyl, thienyl, phenyl, monosubstituted phenyl or di-substituted phenyl, and each ring substituent is fluorine, chlorine, bromine, alkyl having up to four carbon atoms, alkoxy having up to four carbon atoms or trifluoromethyl, with the proviso that said R is always other than hydrogen or alkyl or phenylalkyl when each of said X, Y and Z is hydrogen. These novel compounds are aldose reductase inhibitors and therefore, possess the ability to reduce or inhibit sorbitol accumulation in the lens and peripheral nerves of diabetic subjects.

Of especial interest in this connection are such typical and preferred member compounds of the invention as 1-(p-fluorophenyl)-5-fluoro-*spiro*-[indoline-3,5'-oxazolidine]-2,2',4'-trione, 1-methyl-5-chloro-*spiro*-[indoline-3,5'-oxazolidine]-2,2',4'-trione, 1-isopropyl-5-chloro-*spiro*-[indoline-3,5'-oxazolidine]-2,2',4'-trione and 1-(p-chlorobenzyl)-5-chloro-*spiro*-[indoline-3,5'-oxazolidine]-2,2',4'-trione, respectively. These particular compounds are highly potent as regards their aldose reductase inhibitory activity.

In accordance with the process employed for preparing the novel compounds of this invention, an appropriately substituted isatin starting material of the formula:

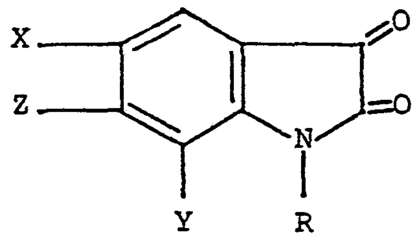

wherein X, Y, Z and R are each as previously defined, is (1) reacted with a lower trialkylsilyl cyanide, such as trimethylsilyl cyanide, to form the corresponding 3-cyano-3-trialkylsilyloxy derivative, followed by treatment with alcoholic hydrogen chloride to yield the desired hydroxy ester which, in turn, is then (2) subjected to the action of chlorosulfonyl isocyanate in tetrahydrofuran and subsequent reductive hydrolysis to yield the corresponding urethane ester, followed by (3) cyclization of the latter ester under non-acidic conditions to ultimately afford the desired spiro-oxindole oxazolidinedione final product of the structural formula previously indicated. The last step of the process is preferably conducted under basic conditions, using a basic catalyst such as potassium carbonate and preferably employing even more strongly basic catalysts such as sodium methoxide or potassium tertiary-butoxide. Suitable solvents for the reaction include reaction-inert organic solvents such as diethyl ether, tetrahydrofuran, dioxane, dimethoxyethane, dimethylformamide and the like. In this way, for example, 1-(p-chlorobenzyl)-5-chloroisatin is converted via 1-(p-chloro-

benzyl)-3-hydroxy-3-carbethoxy-5-chloroindoline-2-one and 1-(p-chlorobenzyl)-3-carbamyloxy-3-carbethoxy-5-chloroindoline-2-one, respectively, to 1-(p-chlorobenzyl)-5-chloro-spiro-[indoline-3,5'-oxazolidine]-2,2',4'-trione.

Compounds of the invention where X and Y are each hydrogen and Z is amino are best prepared by the alkylation of sodio-ethyl oxazolidinedione-5-carboxylate with 2,4-dinitrochlorobenzene, followed by reductive cyclization in a conventional manner. Moreover, compounds of the invention where X and Y are both halogen (as previously defined) and Z is hydrogen may alternatively (and preferably) be prepared from the corresponding unsubstituted compounds wherein at least one of X and Y is hydrogen by means of direct halogenation techniques well known to those skilled in the field of synthetic organic chemistry. Additionally, these same monohalo starting materials (e.g., where X is halogen and Y and Z are both hydrogen) can be converted to the corresponding compounds where Y is nitro and amino, etc., by conventional procedure well-known to those skilled in the art (e.g., nitration and subsequent reduction, etc.)

The ketone starting materials (i.e., carbonyl ring compounds) required for preparing the 3-cyano-3-trialkylsilyloxy derivatives used as intermediates in the first step of the overall process of this invention are, for the most part, known compounds and are either readily available commercially, like isatin (2,3-indolinedione), 1-methylisatin, 1-benzylisatin, 5-fluoroisatin and 5-chloroisatin, etc., or else they can easily be synthesized by those skilled in

the art starting from common chemical reagents and using conventional methods of organic synthesis. For instance, the 1-alkyl-5-haloisatins are easily obtained by alkylating 5-fluoro or 5-chloroisatin with the appropriate alkyl halide of choice in the presence of a base such as potassium carbonate, while the corresponding 1-(p-halophenyl)-5-haloisatins are best synthesized by treatment of the appropriate 4,4'-dihalophenylamine compound with oxalyl chloride, followed by ring-closure with aluminum chloride in the usual manner. In either case, the ultimate starting materials are both readily derived from commercially available compounds.

Inasmuch as the spiro-oxindole oxazolidinedione compounds of this invention all possess one asymmetric center, they may exist in separated d- and l-optically active forms, as well as in racemic or dl-mixtures. The present invention includes all these forms. For instance, an optically active isomer may be obtained by simply resolving the racemic mixture via the use of standard techniques well-known to those skilled in the art, e.g., by fractional crystallization of a spiro-oxindole oxazolidinedione salt derived from an optically active base. Alternatively, the optically active isomers may be prepared by using the appropriate enantiomers as starting materials in the foregoing series of reactions.

The chemical bases which are used as reagents in this invention to prepare the aforementioned pharmaceutically acceptable base salts are those which form non-toxic salts with the herein described acidic spiro-oxindole oxazolidinedione compounds, such as

1-(p-chlorobenzyl)-5-chloro-spiro-[indole-3,5'-oxazolidine]-2,2',4'-trione, for example. These particular non-toxic base salts include those derived from such pharmacologically acceptable cations as sodium, potassium, calcium and magnesium, etc. These salts can easily be prepared by simply treating the aforementioned spiro-oxindole oxazolidinedione compounds with an aqueous solution of the desired pharmacologically acceptable cation, and then evaporating the resulting solution to dryness while preferably being placed under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum production of yields of the desired final product.

As previously indicated, the spiro-oxindole oxazolidinedione compounds of this invention are readily adapted to therapeutic use as aldose reductase inhibitors for the control of chronic diabetic complications, in view of their ability to reduce lens sorbitol levels in diabetic subjects to a statistically significant degree. For instance, 1-(p-chlorobenzyl)-5 chloro-spiro-[indole-3,5'-oxazolidine]-2,2',4'-trione, a typical and preferred agent of the present invention, has been found to inhibit the formation of sorbitol levels in diabetic rats to a significantly high degree

when given by the oral route of administration at dose levels ranging from 0.5 mg./kg. to 20 mg./kg. Furthermore, the herein described compounds of this invention can be administered by either the oral or parenteral routes of administration. In general, these compounds are ordinarily administered in dosages ranging from about 0.10 mg. to about 10 mg. per kg. of body weight per day, although variations will necessarily occur depending upon the weight and condition of the subject being treated and the particular route of administration chosen.

In connection with the use of the spiro-oxindole oxazolidinedione compounds of this invention for the treatment of diabetic subjects, it is to be noted that these compounds may be administered either alone or in combination with pharmaceutically acceptable carriers by either of the routes previously indicated, and that such administration can be carried out in either single or multiple dosages. More particularly, the compounds of this invention can be administered in a wide variety of different dosage forms, i.e., they may be combined with various pharmaceutically-acceptable inert carriers in the form of tablets, capsules, lozenges, troches, hard candies, powders, sprays, aqueous suspensions, injectable solutions, elixirs, syrups, and the like. Such carriers include solid diluents or fillers, sterile aqueous media and various non-toxic organic solvents.

In general, the compounds of the invention will be present in such dosage forms at concentration levels ranging from about 0.5% to about 90% by weight of the total composition to provide the desired unit dosage.

For purposes of oral administration, tablets containing various excipients such as sodium citrate, calcium carbonate and calcium phosphate may be employed along with various disintegrants such as starch and preferably potato or tapioca starch, alginic acid and certain complex silicates, together with binding agents such as polyvinylpyrrolidone, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred materials in this connection would also include the high molecular weight polyethylene glycols. When aqueous suspensions and/or elixirs are desired for oral administration, the essential active ingredient therein may be combined with various sweetening or flavoring agents, coloring matter or dyes, and if so desired, emulsifying and/or suspending agents as well, together with such diluents as water, ethanol, propylene glycol, glycerin and various like combinations thereof.

For purposes of parenteral administration, solutions of these <u>spiro</u>-oxindole oxazolidinediones in sesame or peanut oil or in aqueous propylene glycol or

N,N-dimethylformamide may be employed, as well as sterile aqueous solutions of the corresponding watersoluble, alkali-metal or alkaline-earth metal salts previously enumerated. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal injection purposes. In this connection, the sterile aqueous media employed are all readily obtainable by standard techniques well-known to those skilled in the art. Additionally, it is also possible to administer the aforesaid spiro-oxindole oxazolidinedione compounds topically via an appropriate ophthalmic solution applied dropwise to the eye.

The activity of the compounds of the present invention, as agents for the control of chronic diabetic complications, is determined by their ability to successfully pass one or more of the following standard biological or pharmacological tests, viz., (1) measuring their ability to inhibit the enzyme activity of isolated aldose reductase; (2) measuring their ability to reduce or inhibit sorbitol accumulation in the sciatic nerve of acutely streptozotocinized (i.e., diabetic) rats; (3) measuring their ability to reverse already-elevated sorbitol levels in the sciatic nerve and lens of chronic streptozotocin-induced diabetic rats; (4) measuring their ability to prevent or inhibit galactitol formation in the lens of acutely galactosemic rats, and (5) measuring their ability to delay cataract formation and reduce the severity of lens opacities in chronic galactosemic rats.

PREPARATION A

To a well-stirred suspension consisting of 1.25 g (0.00688 mole) of 5-chloroisatin and 1.9 g (0.01376 mole) of powdered potassium carbonate in 8 ml of dry N,N-dimethylformamide, there were added 2.33 g (0.01445 mole) of p-chlorobenzyl chloride. The resulting mixture was then stirred at room temperature (~25°C) for a period of 20 hours. Upon completion of this step, the spent reaction mixture was diluted with 40 ml of water and then extracted with three separate portions of ethyl acetate. The combined organic extracts were thereafter washed with two separate portions of water and dried over anhydrous magnesium sulfate. After removal of the drying agent by means of filtration and the solvent by means of evaporation under reduced pressure, there was ultimately obtained an orange solid material (3.2 g) as residue. The latter material was then chromatographed over 50 g of silica gel and subsequently eluted with n-hexane to remove excess benzyl chloride, followed by elution with diethyl ether to collect the product. This was accomplished by taking 20 ml fractions and the pure product was isolated in fraction Nos. 20-65, which were thereafter combined and subsequently evaporated to near dryness to yield an orange solid material which consisted of pure 1-(p-chlorobenzyl)-5-chloroisatin. The yield of pure product amounted to 1.3 g (70%). The analytical sample melted at 187-188°C after recrystallization from ethyl acetate. The pure product was further characterized by mass spectroscopy, nuclear magnetic resonance data and infrared absorption spectra, in addition to elemental analysis.

Anal. Calcd. for $C_{15}H_9Cl_2NO_2$: C, 58.85; H, 2.96; N, 4.58.

Found: C, 59.00; H, 3.22; N, 4.68.

PREPARATION B

A mixture consisting of 10.81 g (0.06 mole) of 5-chloroisatin, 13 ml (0.130 mole) of isopropyl iodide and 16.4 g (0.120 mole) of powdered potassium carbonate in 100 ml of dry dimethylformamide was vigorously stirred at room temperature ( ∼25°C) for a period of 20 hours. The reaction mixture was then poured onto water and the desired product was extracted with ethyl acetate. After drying the organic extract over anhydrous magnesium sulfate, the solvent was removed *in vacuo* and the residue crystallized from ethyl acetate to give 7.62 g (57%) of pure 1-isopropyl-5-chloroisatin in the form of orange crystals melting at 144-145°C. The pure product was further characterized by means of mass spectroscopy, nuclear magnetic resonance data and infrared absorption spectra, in addition to elemental analysis.

*Anal*. Calcd. for $C_{11}H_{10}ClNO_2$: C, 59.07; H, 4.51; N, 6.26.
Found: C, 59.17; H, 4.60; N, 6.22.

PREPARATION C

The procedure described in Preparation B was repeated except that methyl iodide was the alkylating agent of choice employed in place of isopropyl iodide, using the same molar proportions as before. In this particular case, the corresponding final product obtained was 1-methyl-5-chloroisatin, mp 170-171.5°C after recrystallization from ethyl acetate. The yield of pure product was 74% of the theoretical value. The pure product was identical in every respect with the prior art compound reported by S. Inaba et al. in *Chem. Pharm. Bull.* (Tokyo), Vol. 24, p. 1076 (1976).

PREPARATION D

To a stirred mixture consisting of 3.0 ml (0.035 mole) of oxalyl chloride and 35 ml of methylene chloride, there was added in a dropwise fashion a clear solution consisting of 5.14 g (0.025 mole) of 4,4'-difluorodiphenylamine [R. I. Walter, Journal of the American Chemical Society, Vol. 77, p. 5999 (1955)] dissolved in 35 ml of methylene chloride. After 40 minutes at room temperature (~25°C), 5.28 g (0.0396 mole) of anhydrous aluminum chloride was added to the mixture in one full portion with the aid of vigorous agitation. Stirring of same was then continued for a period of 45 minutes while at room temperature, after which time the reaction mixture was poured onto ice water and the product extracted with ethyl acetate. After drying the organic extract over anhydrous magnesium sulfate, the solvent was removed in vacuo and the residue crystallized from ethyl acetate/n-hexane to give 4.94 g (76%) of pure 1-(p-fluorophenyl)-5-fluoroisatin in the form of orange crystals melting at 201-202°C. The pure product was further characterized by means of mass spectroscopy, nuclear magnetic resonance data and infrared absorption spectra, in addition to elemental analysis.

Anal. Calcd. for $C_{14}H_7F_2NO_2$: C, 64.87; H, 2.72; N, 5.40.
Found: C, 65.09; H, 2.93; N, 5.38.

PREPARATION E

A mixture consisting of 2.59 g (0.01 mole) of 1-(p-fluorophenyl)-5-fluoroisatin (prepared as described in Preparation D) and 1.6 ml (0.012 mole) of trimethylsilyl cyanide was heated at 130°C for a period of 20 minutes. The resulting clear yellow solution was then diluted with 20 ml of 98% ethanol, cooled to 0°C and saturated with dry hydrogen chloride gas at 0°C. After

ten minutes at room temperature (~ 25°C), the reaction mixture was diluted with ethyl acetate, washed with water and then saturated with aqueous sodium bicarbonate solution. After drying over anhydrous magnesium sulfate, the solvent was removed in vacuo and the residue was subsequently filtered through silica gel using methylene chloride as the eluant. In this manner, there were ultimately obtained 2.60 g (78%) of pure 1-(p-fluorophenyl)-3-hydroxy-3-carbethoxy-5-fluoro-indoline-2-one in the form of white crystals melting at 138-139°C (after crystallization from methylene chloride/ n-hexane). The pure product was further characterized by means of mass spectroscopy, nuclear magnetic resonance data and infrared absorption spectra, in addition to elemental analysis.

Anal. Calcd. for $C_{17}H_{13}F_2NO_3$: C, 61.26; H, 3.93; N, 4.20.
Found: C, 61.34; H, 4.02; N, 4.44.

PREPARATION F

The procedure described in Preparation E was repeated except that 1-isopropyl-5-chloroisatin (prepared as described in Preparation B) was the starting material employed in place of 1-(p-fluorophenyl)-5-fluoroisatin, using the same molar proportions as before. In this particular case, the corresponding final product obtained was 1-isopropyl-3-hydroxy-3-carbethoxy-5-chloroindoline-2-one (in the form of a glass). The yield of pure product amounted to 82% of the theoretical value. The pure product was further characterized by means of mass spectroscopy, nuclear magnetic resonance data and infrared absorption spectra, in addition to exact mass spectral analysis.

Exact Mass: Calcd. for $C_{14}H_{16}ClNO_4$: 299.0739.
Found: C, 299.0721.

PREPARATION G

The procedure described in Preparation E was repeated except that 1-methyl-5-chloroisatin (prepared as described in Preparation C) was the starting material employed in place of 1-(p-fluorophenyl)-5-fluoroisatin, using the same molar proportions as before. In this particular case, the corresponding final product obtained was 1-methyl-3-hydroxy-3-carbethoxy-5-chloroindoline-2-one, mp 121-122°C (after crystallization from ethyl acetate/n-hexane). The yield of pure product amounted to 68% of the theoretical value. The pure product was further characterized by means of mass spectroscopy, nuclear magnetic resonance data and infrared absorption spectra, in addition to elemental analysis.

Anal. Calcd. for $C_{12}H_{12}ClNO_4$: C, 53.44; H, 4.49; N, 5.19. Found: C, 53.45; H, 4.51; N, 5.14.

PREPARATION H

The procedure described in Preparation E was repeated except that 1-(p-chlorobenzyl)-5-chloroisatin (prepared as described in Preparation A) was the starting material employed in place of 1-(p-fluorophenyl)-5-fluoroisatin, using the same molar proportions as before. In this particular case, the corresponding final product obtained was 1-(p-chlorobenzyl)-3-hydroxy-3-carbethoxy-5-chloroindoline-2-one, mp 144-145°C (after crystallization from methylene chloride/n-hexane). The yield of pure product amounted to 82% of the theoretical value. The pure product was further characterized by means of mass spectroscopy, nuclear magnetic resonance data and infrared absorption spectra, in addition to elemental analysis.

Anal. Calcd. for $C_{18}H_{15}Cl_2NO_4$: C, 56.86; H, 3.98; N, 3.68. Found: C, 57.17; H, 4.08; N, 3.72.

PREPARATION I

To a solution consisting of 2.33 g (0.007 mole) of 1-(p-fluorophenyl)-3-hydroxy-3-carbethoxy-5-fluoroindoline-2-one (prepared as described in Preparation E) dissolved in 35 ml of freshly distilled tetrahydrofuran at 0°C, there was added 0.65 ml (0.0075 mole) of chlorosulfonyl isocyanate. After five minutes at 0°C, 15 ml of saturated aqueous sodium sulfite and 15 ml of saturated aqueous sodium bicarbonate were added. After stirring for a period of five minutes while at room temperature (~ 25°C), the reaction mixture was acidified with 3N hydrochloric acid and the desired product extracted with ethyl acetate. The organic layer was then washed with saturated aqueous sodium bicarbonate solution and dried over anhydrous magnesium sulfate, followed by the subsequent removal of the solvent *in vacuo*. In this manner, there were ultimately obtained 2.3 g (87%) of pure 1-(p-fluorophenyl)-3-carbamyloxy-3-carbethoxy-5-fluoroindoline-2-one in the form of white crystals melting at 182-183°C (after crystallization from methylene chloride/n-hexane). The pure product was further characterized by means of mass spectroscopy, nuclear magnetic resonance data and infrared absorption spectra, in addition to elemental analysis.

Anal. Calcd. for $C_{18}H_{14}F_2N_2O_5$: C, 57.45; H, 3.75; N, 7.44.
Found: C, 57.34; H, 3.80; N, 7.44.

PREPARATION J

The procedure described in Preparation I was repeated except that 1-isopropyl-3-hydroxy-3-carbethoxy-5-chloroindoline-2-one (prepared as described in Preparation F) was the starting material employed in place of 1-(p-fluorophenyl)-3-hydroxy-3-carbethoxy-5-fluoroindoline-2-one, using the same molar proportions as before. In this particular case, the corresponding final product obtained was 1-isopropyl-3-carbamyloxy-3-carbethoxy-5-chloroindoline-2-one, mp 244-246°C (after crystallization from ethyl acetate). The yield of pure product amounted to 75% of the theoretical value. The pure product was further characterized by means of mass spectroscopy, nuclear magnetic resonance data and infrared absorption spectra, in addition to elemental analysis.

Anal. Calcd. for $C_{15}H_{17}ClN_2O_5$: C, 52.87; H, 5.03; N, 8.22.
Found: C, 52.80; H, 5.11; N, 7.90.

PREPARATION K

The procedure described in Preparation I was repeated except that 1-methyl-3-hydroxy-3-carbethoxy-5-chloroindoline-2-one (prepared as described in Preparation G) was the starting material employed in place of 1-(p-fluorophenyl)-3-hydroxy-3-carbethoxy-5-fluoroindoline-2-one, using the same molar proportions as before. In this particular case, the corresponding final product obtained was 1-methyl-3-carbamyloxy-3-carbethoxy-5-chloroindoline-2-one, mp 185-186°C (after crystallization from methylene chloride/n-hexane). The yield of pure product amounted to 66% of the theoretical value. The pure product was further characterized by means of mass spectroscopy, nuclear magnetic resonance data and infrared absorption spectra, in addition to elemental analysis.

Anal. Calcd. for $C_{13}H_{13}ClN_2O_5$: C, 49.93; H, 4.19; N, 8.96.
Found: C, 50.05; H, 4.25; N, 8.99.

PREPARATION L

The procedure described in Preparation I was repeated except that 1-(p-chlorobenzyl)-3-hydroxy-5-chloroindoline-2-one (prepared as described in Preparation H) was the starting material employed in place of 1-(p-fluorophenyl)-3-hydroxy-3-carbethoxy-5-fluoroindoline-2-one, using the same molar proportions as before. In this particular case, the corresponding final product obtained was 1-(p-chlorobenzyl)-3-carbamyloxy-3-carbethoxy-5-chloroindoline-2-one, mp 182-183°C (after crystallization from methylene chloride/n-hexane). The yield of pure product amounted to 85% of the theoretical value. The pure product was further characterized by means of mass spectroscopy, nuclear magnetic resonance data and infrared absorption spectra, in addition to elemental analysis.

Anal. Calcd. for $C_{19}H_{16}Cl_2N_2O_5$: C, 53.92; H, 3.81; N, 6.62.
Found: C, 53.91; H, 3.92; N, 6.81.

EXAMPLE 1

To a solution consisting of 752 mg (0.002 mole) of 1-(p-fluorophenyl)-3-carbamyloxy-3-carbethoxy-5-fluoroindoline-2-one (prepared as described in Preparation A) dissolved in 9 ml of freshly distilled tetrahydrofuran and 1 ml of dry dimethylformamide at 0°C, there was added 246 mg (0.002 mole) of potassium tert.-butoxide. The resulting mixture was then stirred at 50°C for a period of five minutes. After cooling to 0°C, the reaction mixture was acidified with 3N hydrochloric acid, then diluted with water and finally extracted with diethyl ether/ethyl acetate (2:1 by volume). The desired oxazolidinedione final product was subsequently extracted from the organic layer with dilute aqueous sodium bicarbonate solution. The resulting aqueous bicarbonate layer was thereafter acidified with 3N

hydrochloric acid and the desired final product extracted therefrom with ethyl acetate. After drying the organic extract over anhydrous magnesium sulfate, the solvent was removed in vacuo and the residue crystallized from methylene chloride/n-hexane to ultimately afford 267 mg (54%) of pure 1-(p-fluorophenyl)-5-fluoro-spiro-[indoline-3,5-oxazolidine]-2,2',4'-trione in the form of white crystals melting at 186-187°C. The pure product was further characterized by means of mass spectroscopy, nuclear magnetic resonance data and infrared absorption spectra, in addition to elemental analysis.

Anal. Calcd. for $C_{16}H_8F_2N_2O_2$: C, 58.91; H, 2.44; N, 8.48. Found: C, 58.12; H, 2.63; N, 8.35.

EXAMPLE 2

The procedure described in Example 1 was repeated except that 1-isopropyl-3-carbamyloxy-3-carbethoxy-5-chlorindoline-2-one (prepared as described in Preparation J) was the starting material employed in place of 1-(p-fluorophenyl)-3-carbamyloxy-3-carbethoxy-5-fluoroindoline-2-one, using the same molar proportions as before. In this particular case, the corresponding final product obtained was 1-isopropyl-5-chloro-spiro-[indoline-3,5'-oxazolidine]-2,2',4'-trione, mp 186-187°C (after crystallization from methylene chloride/n-hexane). The yield of pure product amounted to 48% of the theoretical value. The pure product was further characterized by means of mass spectroscopy, nuclear magnetic resonance data and infrared absorption spectra, in addition to elemental analysis.

Anal. Calcd. for $C_{13}H_{11}ClN_2O_4$: C, 52.98; H, 3.76; N, 9.51. Found: C, 52.75; H, 3.81; N, 9.28.

EXAMPLE 3

The procedure described in Example 1 was repeated except that 1-methyl-3-carbamyloxy-3-carbethoxy-5-chloroindoline-2-one (prepared as described in Preparation K) was the starting material employed in place of 1-(p-fluorophenyl)-3-carbamyloxy-3-carbethoxy-5-fluoroindoline-2-one, using the same molar proportions as before. In this particular case, the corresponding final product obtained was 1-methyl-5-chloro-spiro-[indoline-3,5'-oxazolidine]-2,2',4'-trione, mp 285-286°C (after crystallization from ethyl acetate/n-hexane). The yield of pure product amounted to 49% of the theoretical value. The pure product was further characterized by means of mass spectroscopy, nuclear magnetic resonance data and infrared absorption spectra, in addition to elemental analysis.

Anal. Calcd. for $C_{11}H_7ClN_2O_4$: C, 49.55; H, 2.65; N, 10.51. Found: C, 49.59; H, 2.86; N, 10.36.

EXAMPLE 4

The procedure described in Example 1 was repeated except that 1-(p-chlorobenzyl)-3-carbamyloxy-3-carbethoxy-5-chloroindoline-2-one (prepared as described in Preparation L) was the starting material employed in place of 1-(p-fluorophenyl)-3-carbamyloxy-3-carbethoxy-5-fluoro-indoline-2-one, using the same molar proportions as before. In this particular case, the corresponding final product obtained was 1-(p-chlorobenzyl)-5-chloro-spiro-[indoline-3,5'-oxazolidine]-2,2',4'-trione, mp 208-209°C (after crystallization from ethyl acetate/n-hexane). The yield of pure product amounted to 39% of the theoretical value. The pure product was further characterized by means of mass spectroscopy, nuclear magnetic resonance data and infrared absorption spectra, in addition to elemental analysis.

Anal. Calcd. for $C_{17}H_{10}Cl_2N_2O_4$: C, 54.13; H, 2.67; N, 7.43. Found: C, 54.52; H, 2.91; N, 7.63.

EXAMPLE 5

The following spiro-oxindole oxazolidinediones may be prepared by employing the procedures described in the previous examples (as well as Preparations A-L), starting from readily available materials in each instance:

1-phenyl-spiro-[indoline-3,5'-oxazolidine]-2,2',4'-trione

1-(p-chlorophenyl)-5-chloro-spiro-[indoline-3,5'-oxazolidine]-2,2',4'-trione

1-methyl-5-fluoro-spiro-[indoline-3,5'-oxazolidine]-2,2',4'-trione

1-ethyl-5-chloro-spiro-[indoline-3,5'-oxazolidine]-2,2',4'-trione

1-(n-butyl)-5-chloro-spiro-[indoline-3,5'-oxazolidine]-2,2',4'-trione

1-benzyl-5-chloro-spiro-[indoline-3,5'-oxazolidine]-2,2',4'-trione

1-(p-fluorobenzyl)-5-fluoro-spiro-[indoline-3,5'-oxazolidine]-2,2',4'-trione

1-(3,4-dichlorobenzyl)-spiro-[indoline-3,5'-oxazolidine]-2,2',4'-trione

1-[ -(p-chlorophenyl)isopropyl]-5-chloro-spiro-[indoline-3,5'-oxazolidine]-2,2',4'-trione

EXAMPLE 6

The sodium salt of 1-(p-chlorobenzyl)-5-chloro-spiro-[indoline-3,5'-oxazolidine]-2,2',4'-trione may be prepared by dissolving said compound in water containing an equivalent amount in moles of sodium hydroxide and then freeze-drying the mixture. In this way, the desired alkali metal salt of the oxazolidinedione is obtained in the form of an amorphous powder

which is freely-soluble in water.

In like manner, the potassium and lithium salts are also similarly prepared, as are the other alkali metal salts of all the other spiro-oxindole oxazolidinediones of this invention which are reported earlier in Examples 1-3 and 5, respectively.

EXAMPLE 7

The calcium salt of 1-(p-chlorobenzyl)-5-chloro-spiro-[indoline-3,5'-oxazolidine]-2,2',4'-trione may be prepared by dissolving said compound in water containing an equivalent amount in moles of calcium hydroxide and then freeze-drying the mixture. The corresponding magnesium salt is also prepared in like manner, as are all the other alkaline-earth metal salts not only of this particular compound, but also of those spiro-oxindole oxazolidinediones previously described in Examples 1-3 and 5, respectively.

EXAMPLE 8

A dry solid pharmaceutical composition may be prepared by blending the following materials together in the proportions by weight specified below:

| | |
|---|---|
| 1-(p-Chlorobenzyl)-5-chloro-spiro-[indoline-3,5'-oxazolidine]-2,2',4'-trione | 50 |
| Sodium citrate | 25 |
| Alginic acid | 10 |
| Polyvinylpyrrolidone | 10 |
| Magnesium stearate | 5 |

After the dried compound is thoroughly blended, tablets are punched from the resulting mixture, each tablet being of such size that it contains 200 mg of the active ingredient. Other tablets are also prepared in a similar fashion containing 25, 50 and 100 mg of the active ingredient, respectively, by merely using the appropriate amount of the oxazolidinedione compound in each case.

EXAMPLE 9

A dry solid pharmaceutical composition may be prepared by combining the following materials together in the proportions by weight indicated below:

1-(p-Fluorophenyl)-5-fluoro-spiro-[indoline-3,5'-oxazolidine]-2,2',4'-trione....................50
Calcium carbonate...............................20
Polyethylene glycol, average molecular weight 4000....................................30

The dried solid mixture so prepared is then thoroughly agitated so as to obtain a powdered product that is completely uniform in every respect. Soft elastic and hard-filled gelatin capsules containing this pharmaceutical composition are then prepared, employing a sufficient quantity of material in each instance so as to provide each capsule with 250 mg of the active ingredient.

EXAMPLE 10

The following spiro-oxindole oxazolidinedione final products of Examples 1-4, respectively, were tested for their ability to reduce or inhibit aldose reductase enzyme activity via the procedure of S. Hayman et al., as described in the Journal of Biological Chemistry, Vol 240, p. 877 (1965) and as modified by K. Sestanj et al. in U.S. Patent No. 3,821,383. In every case, the substrate employed was partially purified aldose reductase enzyme obtained from calf lens. The results obtained with each compound are expressed below in terms of percent inhibition of enzyme activity with respect to the various concentration levels tested:

| Compound | Percent Inhibition (%) $10^{-4}$M | $10^{-5}$M | $10^{-6}$M | $10^{-7}$M |
|---|---|---|---|---|
| Product of Example 1 | - | 87 | 65 | 25 |
| Product of Example 2 | - | 80 | 45 | 17 |
| Product of Example 3 | - | 67 | 31 | 10 |
| Product of Example 4 | 100 | 89 | 81 | 60 |

CLAIMS

1. A compound selected from the group consisting of spiro-oxindole derivatives of the formula:

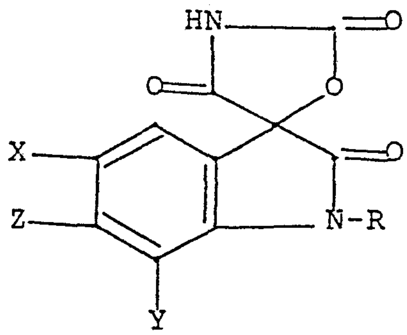

and the base salts thereof with pharmacologically acceptable cations, wherein

X and Y are each hydrogen, fluorine, chlorine, bromine, nitro or amino;

Z is hydrogen or amino, with the proviso that Z is hydrogen when at least one of X and Y is other than hydrogen; and

R is hydrogen, alkyl having from one to four carbon atoms, aryl or aralkyl having up to three carbon atoms in the alkyl moiety wherein each of said aryl moieties is naphthyl, pyridyl, furyl, thienyl, phenyl, monosubstituted phenyl or di-substituted phenyl, and each ring substituent is fluorine, chlorine, bromine, alkyl having up to four carbon atoms, alkoxy having up to four carbon atoms or trifluoromethyl, with the proviso that said R is always other than hydrogen or alkyl or phenylalkyl when each of said X, Y and Z is hydrogen.

2. A compound as claimed in claim 1 wherein X is fluorine, Y and Z are each hydrogen and R is mono-substituted phenyl.

3. A compound as claimed in claim 2 wherein R is p-fluorophenyl.

4. A compound as claimed in claim 1 wherein X is chlorine, Y and Z are each hydrogen and R is alkyl having from one to four carbon atoms, or mono-substituted phenylalkyl having up to three carbon atoms in the alkyl moiety.

5. A compound as claimed in claim 4 wherein R is p-chlorobenzyl.

6. 1-(p-Fluorophenyl)-5-fluoro-spiro-[indoline-3,5'-oxazolidine]-2,2',4'-trione.

7. 1-Methyl-5-chloro-spiro-[indoline-3,5'-oxazolidine]-2,2',4'-trione.

8. 1-Isopropyl-5-chloro-spiro-[indoline-3,5'-oxazolidine]-2,2',4'-trione.

9. 1-(p-Chlorobenzyl)-5-chloro-spiro-[indoline-3,5'-oxalidine]-2,2',4'-trione.

10. A pharmaceutical composition suitable for oral administration comprising a pharmaceutically acceptable carrier and a compound as claimed in claim 1 in an amount effective for the treatment of diabetes-associated chronic complications.

CLAIMS FOR AUSTRIA (AT)

1. An analogy process for preparing spiro-oxindole oxazolidinedione derivatives of the formula:

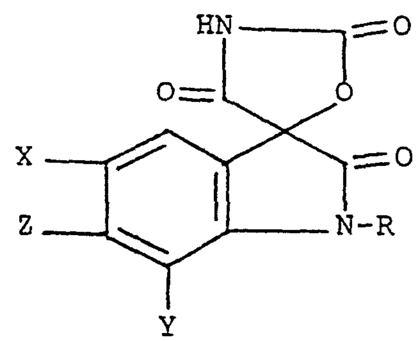

and the base salts thereof with pharmacologically acceptable cations, wherein X and Y are each hydrogen, fluorine, chlorine, bromine, nitro or amino; Z is hydrogen or amino, with the proviso that Z is hydrogen when at least one of X and Y is other than hydrogen; and R is hydrogen, alkyl having from one to four carbon atoms, aryl or aralkyl having up to three carbon atoms in the alkyl moiety wherein each of said aryl moieties is naphthyl, pyridyl, furyl, thienyl, phenyl, monosubstituted phenyl or di-substituted phenyl, and each ring substituent is fluorine, chlorine, bromine, alkyl having up to four carbon atoms, alkoxy having up to four carbon atoms or trifluoromethyl, with the proviso that said R is always other than hydrogen or alkyl or phenylalkyl when each of said X, Y and Z is hydrogen, characterized by (a) reacting an appropriately substituted isatin starting material of the formula:

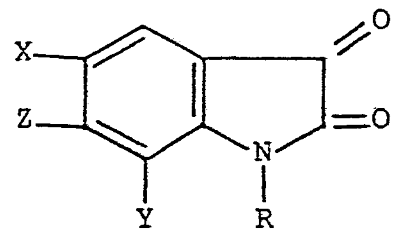

wherein X, Y Z and R are each as previously defined, with a lower trialkylsilyl cyanide to form the corresponding 3-cyano-3-trialkylsilyloxy derivative, followed by treatment of the latter derivative with alcoholic hydrogen chloride to yield the desired hydroxy ester; then (b) subjecting the latter ester to the action of chlorosulfonyl isocyanate in tetrahydrofuran, followed by reductive hydrolysis to yield the corresponding urethane ester; and thereafter (c) cyclizing the latter ester under non-acidic conditions to ultimately afford the desired *spiro*-oxindole oxazolidinedione final product of the structural formula previously indicated; and, if desired, converting said *spiro*-oxindole oxazolidinedione final product to a pharmacologically acceptable base salt thereof.

2. A process as claimed in claim 1, characterized by the fact that the cyclization reaction in part (c) is carried out in a reaction-inert organic solvent in the presence of a strongly basic catalyst.

3. A process as claimed in claim 3, characterized by the fact that said solvent is dimethylformamide and said basic catalyst is potassium *tertiary*-butoxide.

4. A process as claimed in claim 1 wherein the *spiro*-oxindole oxazolidinedione derivative prepared is 1-(*p*-fluorophenyl)-5-fluoro-*spiro*-[indoline-3,5'-oxazolidine]-2,2',4'-trione.

5. A process as claimed in claim 1 wherein the spiro-oxindole oxazolidinedione derivative prepared is 1-methyl-5-chloro-spiro-[indoline-3,5'-oxazolidine]-2,2',4'-trione.

6. A process as claimed in claim 1 wherein the spiro-oxindole oxazolidinedione derivative prepared is 1-isopropyl-5-chloro-spiro-[indoline-3,5'-oxazolidine]-2,2',4'-trione.

7. A process as claimed in claim 1 wherein the spiro-oxindole oxazolidinedione derivative prepared is 1-(p-chlorobenzyl)-5-chloro-spiro-[indoline-3,5'-oxazolidine]-2,2',4'-trione.

European Patent Office

# EUROPEAN SEARCH REPORT

0079675

Application number

EP 82 30 5385

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | DE-A-2 933 441 (PFIZER INC.) * Claims 1-3 * & US - A - 4 200 642 (Cat. A) | 1-10 | C 07 D 498/10<br>A 61 K 31/42<br>//(C 07D 498/10<br>263/00,209/00 |
| A | JOURNAL OF THE CHEMICAL SOCIETY, October 1965 J.W. CLARK-LEWIS et al. "Reactions of alloxan witharomatic amines; dioxindole-3-carboxyureides and oxidole-3-spiro-5'-oxazolidine-2', 4'-diones" pages 5551-5556 * Page 5553, formula IX; page 5555, lines 9-39 * | 1-9 | |
| | | | TECHNICAL FIELDS SEARCHED (Int. Cl. 3) |
| | | | A 61 K 31/42<br>C 07 D 498/10 |

The present search report has been drawn up for all claims

| Place of search | Date of completion of the search | Examiner |
|---|---|---|
| BERLIN | 06-01-1983 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document